# FASCICULE DE BREVET EUROPEEN

(11) **EP 2 067 033 B1**
(45) Date de publication et mention de la délivrance du brevet: **31.07.2013**
(21) Numéro de dépôt: 07823392.1
(22) Date de dépôt: 02.08.2007
(51) Int. Cl.: G01N 33/28, G01N 21/85, G01N 21/88

(54) **PROCÉDÉ DE MISE EN SÉCURITÉ DES ORGANES DU GROUPE MOTOPROPULSEUR D'UN VÉHICULE À LA SUITE D'UNE DÉGRADATION DU CARBURANT**
VERFAHREN ZUR GEWÄHRLEISTUNG DER SICHERHEIT DER BESTANDTEILE DER ANTRIEBSKETTE EINES FAHRZEUGS NACH VERSCHLECHTERUNG DES BRENNSTOFFS
METHOD FOR ENSURING THE SAFETY OF THE COMPONENTS OF THE DRIVE TRAIN OF A VEHICLE FOLLOWING THE DETERIORATION OF THE FUEL

(30) Priorité: 21.08.2006 FR 0607420
(43) Date de publication de la demande: 10.06.2009
(73) Titulaire: SP3H, 13100 Aix-en-Provence (FR)
(72) Inventeur: LUNATI, Alain, F-13580 La Fare les Oliviers (FR); FOURNEL, Johan, F-84440 Robion (FR)
(74) Mandataire: Breesé, Pierre
(86) Numéro de dépôt international: PCT/FR2007/001339
(87) Numéro de publication internationale: WO 2008/023104

(56) Documents cités:
- DE-A1- 3 435 706
- DE-A1- 10 251 837
- DE-A1- 10 349 741
- DE-A1-102005 001 716
- FR-A1- 2 628 836
- US-A- 4 770 129
- US-A1- 2002 144 456

## Description

L'invention concerne un procédé préventif de mise en sécurité des organes du groupe motopropulseur d'un véhicule équipé d'un moteur thermique, avant ou pendant sa phase de démarrage à la suite d'une modification (dégradation, pollution) de la nature du carburant contenu dans le réservoir et le système d'alimentation en carburant du moteur.

Malgré les dispositions réglementaires ou internes, prises par les distributeurs de carburants et les constructeurs de véhicules, telles que les procédures qualités des raffineurs et distributeurs, l'affichage de la nature des carburants en station, le diamètre du bec de pistolet de distribution et le diamètre du système de remplissage du réservoir en particulier, de nombreux utilisateurs introduisent volontairement ou non un carburant non adapté dans le réservoir de leur véhicule. Un nombre croissant de véhicules est utilisé avec des produits non agréés par les constructeurs et les services de douanes, comme des huiles de fritures usagées, des huiles végétales non estérifiées, des fiouls domestiques provoquant des dégât importants pour le groupe motopropulseur, son système d'alimentation en carburant et son système de post-traitement. Les dégradations (encrassage des injecteurs, du moteur, du réservoir, bouchage des filtres, grippage des pompes, désactivation des catalyseurs) peuvent être sévères, impactent lourdement les phases d'injections et de combustions du moteur et augmentent les émissions polluantes réglementées ou non, et peuvent mener à la casse moteur. De même certains carburants tels que les émulsions eau/gazole ou essence/alcool ou gazole/biocarburants peuvent être instables et leur qualité se dégrader dans le temps (stabilité au stockage, phénomène de démixtion entre l'essence et l'éthanol ou le gazole et le diester au dessus de 5%). Ces diverses sources de dégradation de la nature du carburant entraînent potentiellement un accroissement de la pollution du véhicule, des dommages pour le véhicule ou tout au moins des opérations de corrections importantes.

US-A-4 770 129 divulgue un capteur pour déterminer le ratio entre le gazole et l'alcool, utilisés comme carburant.

DE 10 2005 001 716 divulgue un capteur d'identification du carburant présent dans le réservoir.

L'invention vise à répondre au besoin de mise en sécurité préventive des organes du groupe motopropulseur d'un véhicule équipé d'un moteur thermique, avant ou pendant sa phase de démarrage à la suite d'une dégradation de la nature du carburant contenu dans le réservoir et le système d'alimentation en carburant. La nature et le niveau de la dégradation sont mesurés par un système de type microanalyseur basé sur la mesure des interactions entre un rayonnement électromagnétique et les molécules comme par exemple le Carbone, l'Hydrogène, l'Oxygène qui constituent le carburant. Ce système est connecté à un système actif ou passif visant à informer l'utilisateur de manière visuelle ou sonore et/ou à mettre automatiquement en sécurité des organes du groupe motopropulseur.

L'innovation permet de répondre à un problème connu et récurrent dont le niveau va croissant car elle permet d'alerter l'utilisateur et/ou de stopper préventivement le processus de démarrage du véhicule afin de confiner au système d'alimentation du véhicule et à lui seul l'impact de la dégradation de la nature du carburant. Une simple vidange et rinçage du réservoir suffira à remettre le véhicule en condition opérationnelle.

A cet effet, l'invention concerne un procédé de mise en sécurité des organes du groupe motopropulseur d'un véhicule équipé d'un moteur thermique, avant ou pendant sa phase de démarrage à la suite d'une dégradation de la nature du carburant contenu dans le réservoir et le système d'alimentation en carburant du moteur, iled*-pmoedé-étemt selon la revendication 1.

Le système d'analyse est constitué d'au moins un microanalyseur implanté dans le circuit carburant comprenant le système de remplissage, le réservoir, les pompes, les filtres à carburant, le circuit d'alimentation du moteur et le circuit retour vers le réservoir.

Les exemples suivants illustrent certaines des actions visant à la mise en sécurité des organes du groupe motopropulseur, ces actions pouvant être mises en oeuvre au cours de l'étape d'activation du système de mise en sécurité des organes du groupe motopropulseur :
- Alarme de l'utilisateur de manière sonore ou visuelle,
- Activation automatique d'un système empêchant le démarrage du véhicule,
- Activation automatique d'un système de purge du ou des filtres à carburant,
- Activation automatique d'un système permettant le by-pass des filtres post-traitement d'échappement.

Les exemples suivants illustrent certaines sources possibles de dégradations ou de pollutions possibles du carburant :
- Essence introduite dans le réservoir d'un véhicule Diesel,
- Gazole introduit dans le réservoir d'un véhicule essence,
- Fioul Domestique introduit dans le réservoir d'un véhicule essence ou Diesel,
- Autres produits introduits dans le réservoir d'un véhicule essence ou Diesel, notamment :
   o Produits présentant une haute teneur en soufre,
   o Produits présentant une teneur importante en molécules d'eau libre,
- Émulsion eau /gazole dégradée,
- Huile de friture usagée ou non,
- Huile végétale non estérifiée (huile de table),
- Démixtion (séparation) Essence/Ethanol,
- Démixtion Gazole/EMHV.

Selon une réalisation particulière, la mesure des interactions entre un rayonnement électromagnétique et les molécules constituants le carburant par le système d'analyse comprend une étape d'analyse spectroscopique des hydrocarbures composant le carburant. L'analyse spectroscopique consiste en une analyse proche infrarouge du carburant.

En effet, l'analyse proche infrarouge est particulièrement bien adaptée au diagnostic de la dégradation de la nature des carburants dans le sens ou l'analyse proche infrarouge est une méthode très sensible et que le spectre proche infrarouge peut être considéré comme « l'ADN » du produit. De plus, l'analyse proche infrarouge est particulièrement répétable.

Il est possible de citer les ouvrages de référence pour le proche infrarouge comme celui de L. G. WEYER publié en 1985 ou le « Handbook of near infrared analysis » publié en 1992 ou des publications plus spécifiques comme les applications spectroscopiques en pétrochimie et raffinage comme présentées dans les articles de Jérôme WORKMAN Jr en 1996 ou de M. VALLEUR en 1999.

D'autres objets et avantages de l'invention apparaîtront au cours de la description qui suit, en référence aux figures annexées.
La figure 1 est une représentation schématique d'un circuit d'alimentation en carburant d'un moteur dans lequel le procédé selon l'invention est mis en oeuvre avec un premier mode de réalisation du microanalyseur du système d'analyse.
La figure 2 est une représentation schématique similaire à la figure 1 avec un deuxième mode de réalisation du microanalyseur du système d'analyse.
La figure 3 est une représentation schématique d'un circuit d'alimentation en carburant d'un moteur dans lequel les différentes possibilités de positionnement du système d'analyse du procédé selon l'invention sont mises en évidence.
La figure 4 est un diagramme représentant les étapes principales du procédé.
La figure 5 représente deux méthodologies de mise en évidence de dégradation du carburant.

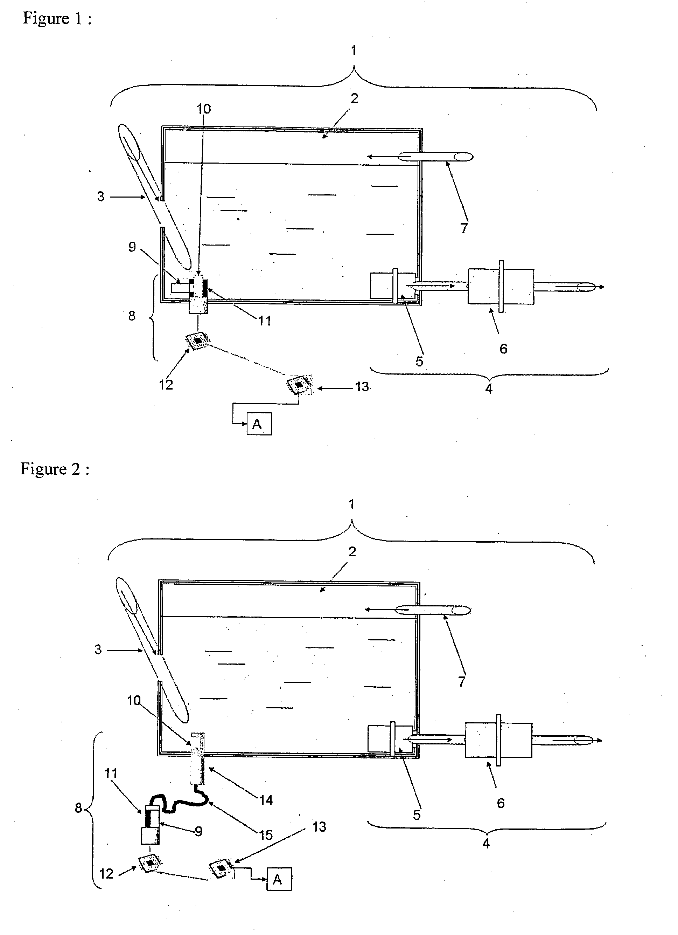

En référence à la figure 1, on décrit un procédé de mise en sécurité des organes du groupe motopropulseur d'un véhicule équipé d'un moteur thermique, avant ou pendant sa phase de démarrage du moteur utilisant un système d'analyse comprenant un microanalyseur 8 de diagnostic de dégradation de la nature du carburant contenu dans le réservoir et le système d'alimentation en carburant du moteur.

Le moteur est alimentée en carburant par le circuit carburant 1, comprenant un réservoir 2, un système de remplissage du réservoir 3 et un circuit d'alimentation en carburant 4. Le circuit comprend par exemple une ou plusieurs pompes à carburant 5, un ou plusieurs filtres à carburant 6 et le circuit retour vers le réservoir 7. Le procédé selon l'invention est adapté pour tout type de carburants (gaz, gaz liquéfié, essence, kérosène, gazole, émulsion eau/gazole, fiouls, biocarburants) répondant aux normes sur les carburants et biocarburants, additivés ou pas, dont les constituants majeurs sont le Carbone, l'Hydrogène et l'Oxygène.

Le diagnostic de dégradation de la nature du carburant contenu dans le réservoir et le système d'alimentation en carburant du moteur consiste en une analyse proche infrarouge du carburant. Elle pourrait également consister en une analyse par Infrarouge, ou une analyse chromatographique en phase gaz ou liquide ou une analyse RMN ou une analyse par ultraviolet ou en plusieurs de ces analyses conduites simultanément selon le même principe.

Selon une réalisation représentée sur la figure 1, un microanalyseur spectroscopique 8 est implanté dans le circuit carburant 1 et est relié à un système électronique ou numérique de mise en sécurité 13 des organes du groupe motopropulseur de manière active ou passive (A). Le système de mise en sécurité 13 est un système actif ou passif informant le calculateur moteur.

Dans le cas d'une analyse proche infrarouge, le microanalyseur 8 est constitué d'une source lumineuse 9, d'un système de séparation de lumière, d'une cellule d'échantillonnage du carburant 10, d'un système de détection photosensible 11 et d'un calculateur dédié 12. Le calculateur dédié 12 permet de piloter les séquences de mesure, de régler et de contrôler le bon fonctionnement du microanalyseur 8. Le calculateur 12 contient les modèles permettant d'effectuer la totalité des calculs associés au traitement du spectre proche infrarouge. Le calculateur 12 est relié au système électronique ou numérique de mise en sécurité 13 des organes du groupe motopropulseur de manière active ou passive.

Dans le cas du proche infrarouge, le microanalyseur 8 peut comporter indifféremment une seule source et un seul détecteur ou plusieurs sources lumineuses et un seul détecteur ou une seule source et plusieurs détecteurs ou plusieurs sources lumineuses et plusieurs détecteurs. Il peut utiliser dans le cas du proche infrarouge non dispersif des filtres interférentiels ou à cristal ou un système à transformée de fourier. Le microanalyseur 8 peut être à accès séquentiels ou multiplexés.

Selon une autre réalisation représentée sur la figure 2, l'utilisation de fibres optiques 15 et d'une sonde plongeante 14 adaptées est possible pour délocaliser le système d'échantillonnage des autres composants du microanalyseur 8.

Le microanalyseur 8 peut être un spectromètre proche infrarouge à barrette composées d'une pluralité de photodiodes qui émettent chacune l'intensité lumineuse à une longueur d'onde donnée. Le détecteur 11 est un semi-conducteur à base de Silicium (Si) ou d'un alliage de type complexe (InGaAs, InAs, InSb, PbS, PbSe) à haute sensibilité. Le détecteur 11 peut être refroidi ou non.

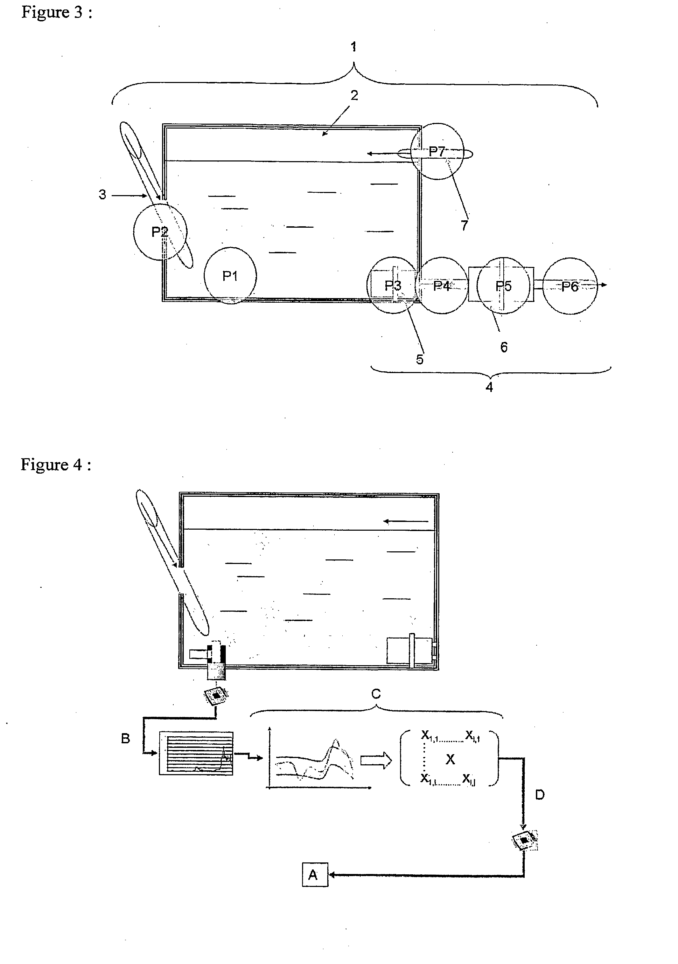

La figure 3 montre que le microanalyseur 8 peut être placé dans le réservoir (Position P1), au niveau du système de remplissage du réservoir (Position P2), dans le circuit d'alimentation en carburant du moteur 4. Dans ce dernier cas, le microanalyseur 8 peut être placé dans la pompe (Position P3) entre la pompe 5 et le filtre 6 (Position P4), dans le filtre 6 (Position P5) ou après le filtre 6 (Position P6). Le microanalyseur peut être également implanté dans le circuit retour 7 du carburant (Position P7).

Le microanalyseur 8 est agencé pour effectuer des mesures dans les régions spectrales comprises entre 780 et 2500 nanomètres (12820 cm-¹ à 4000 cm⁻¹). On peut par exemple prévoir des plages de mesures successives comprises entre 780 nanomètres et 1100 nanomètres (12820 cm⁻¹ à 9090 cm⁻¹), 1100 nanomètres et 2000 nanomètres (9090 cm⁻¹ à 5000 cm⁻¹) et 2000 nanomètres et 2500 nanomètres (5000 cm⁻¹ à 4000 cm⁻¹). A cet effet, le système d'échantillonnage est agencé pour présenter un trajet optique, c'est-à-dire une épaisseur de la cellule de mesure au travers de laquelle se fait la mesure, comprise entre 0,5 millimètres et 100 millimètres, c'est-à-dire des trajets optiques correspondant aux plages de longueurs d'ondes de 50 millimètres à 100 millimètres dans le premier cas, de 10 millimètres à 20 millimètres dans le second cas et de 0.5 millimètres à 5 millimètres dans le dernier cas.

Le microanalyseur 8 est agencé pour effectuer le spectre proche infrarouge du carburant circulant dans le circuit carburant d'alimentation 1 du moteur en réflectance, transmittance, absorbance ou en diffusion.

Le microanalyseur (8) possède une résolution spectacle (précision) réglable de 1 cm⁻¹ à 20 cm⁻¹ préférentiellement à 4 cm⁻¹.

Le système optique et d'échantillonnage du microanalyseur 8 peut être également autonettoyant ce qui permet d'éviter d'avoir à le démonter afin de le nettoyer.

La figure 4 représente les différentes étapes du procédé :
- B : Collecte du spectre proche infrarouge ;
- C : Méthode mathématique permettant de mettre en évidence une dégradation de la nature du carburant, de son type et niveau ;
- D : Transfert de la table d'adressage du calculateur (12) du microanalyseur (8) au système de mise en sécurité 13 active ou passive des organes du groupe motopropulseur.
- A : Mise en sécurité de manière active ou passive des organes du groupe motopropulseur.

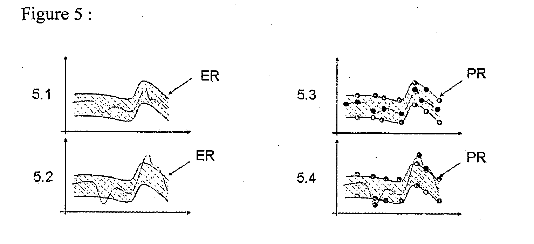

La figure 5 illustre la méthode mathématique de détermination de la dégradation de la nature du carburant. Les mesures des spectres en proche infrarouge du carburant sont faites par exemple en absorbance dans les zones de longueurs d'onde considérées. Les valeurs des absorbances mesurées à chaque longueur d'onde sélectionnée sont comparées à une ou des enveloppes spectrales (ER) (5.1 et 5.2) ou points de référence (PR) (5.3 et 5.4) de manière à déterminer l'existence d'une dégradation de la nature du carburant, et plus précisément le type et le niveau (échelle) de cette dégradation. Dans les exemples 5.1 et 5.3, les valeurs des absorbances du carburant mesurées à chaque longueur d'onde sélectionnée sont comprises dans les enveloppes (ER) ou points de références (PR), ceci permet de conclure à l'absence de dégradation notable du carburant. Dans les exemples 5.2 et 5.4, les valeurs des absorbances du carburant mesurées à chaque longueur d'onde sélectionnée ne sont pas comprises dans les enveloppes (ER) ou points de références (PR), ceci permet de conclure à l'existence d'une dégradation notable du carburant ; le type de dégradation peut être déterminé par l'étude des longueurs d'onde dont les absorbances sont en dehors des enveloppes ou points de références ; les écarts entre les absorbances mesurées à chaque longueur d'onde sélectionnée et les enveloppes ou points de références permettent de déterminer le niveau (échelle) de cette dégradation. Le système comporte de plus des moyens d'autodiagnostic permettant de valider automatiquement le ou les résultats ou de mettre en évidence de manière automatique le défaut de fonctionnement du système. Les moyens d'autodiagnostic permettent d'assurer le bon fonctionnement du système ou le cas échéant pour informer l'utilisateur, l'EOBD et le contrôle moteur. Le ou les résultats sont envoyés vers le ou les systèmes de mise en sécurité 13 active ou passive des organes du groupe motopropulseur.

Un exemple de table (figure 3 [X]) adressée par le calculateur 12 du microanalyseur 8 au système de mise en sécurité 13 active ou passive des organes du groupe motopropulseur est représenté dans le tableau ci-dessous. La table est celle obtenue pour une dégradation de la nature du carburant due à l'introduction d'eau.

| Dégradation | Type de dégradation | Niveau de dégradation | Statut autodiagnostic |
|---|---|---|---|
| Oui | Eau | 3 | OK |

Dans le cas ou une dégradation de la nature du carburant est détectée, le système numérique ou électronique 13 peut mettre automatiquement des organes du groupe motopropulseur en sécurité ou informer de manière visuelle ou sonore l'utilisateur ; ceci dans le but d'éviter d'endommager le groupe motopropulseur.

Une étape de stockage des dégradation de la nature des carburant, de leur type et niveau est utilisée de façon à former un historique précis de ces dégradations.

Le système actif de mise en sécurité 13 peut agir directement ou indirectement sur les paramètres du circuit carburant, les paramètres d'injection de carburant, les paramètres de combustion, les paramètres de post traitement et/ou les paramètres de démarrage du véhicule.

## Revendications

1. Procédé de mise en sécurité des organes du groupe motopropulseur d'un véhicule équipé d'un moteur thermique, avant ou pendant sa phase de démarrage à la suite d'une dégradation de la nature du carburant contenu dans le réservoir (2) et le système d'alimentation en carburant du moteur, ledit procédé comportant une étape de diagnostic du type et de l'importance de la dégradation de la nature du carburant, ladite étape étant basée sur la mesure des interactions entre un rayonnement électromagnétique et les molécules constituants le carburant, ladite mesure étant réalisée par un système d'analyse, et une étape d'activation d'un système de mise en sécurité (13) des organes du groupe motopropulseur en fonction des résultats de l'étape d'analyse, la mesure des interactions entre des radiations électromagnétiques et les molécules composant le carburant consistant en une analyse spectroscopique infrarouge et/ou une analyse spectroscopique ultraviolet et/ou une analyse spectroscopique RMN, les valeurs des absorbances, transmittances, réflectances ou diffusions mesurées à chaque longueur d'onde sélectionnée étant comparées à une ou des enveloppes spectrales ou points de référence de manière à déterminer l'existence d'une dégradation de la nature du carburant, et plus précisément le type et le niveau de cette dégradation.

2. Procédé selon la revendication 1, **caractérisé en ce que** le système de mise en sécurité est un système d'alarme visuelle ou sonore de l'utilisateur.

3. Procédé selon la revendication 1, **caractérisé en ce que** le système de mise en sécurité est un système actif ou passif informant le calculateur moteur.

4. Procédé selon la revendication 3, **caractérisé en ce que** le système actif de mise en sécurité agit directement ou indirectement sur les paramètres du circuit carburant, les paramètres d'injection de carburant, les paramètres de combustion, les paramètres de post traitement et/ou les paramètres de démarrage du véhicule.

5. Procédé selon la revendication 1, **caractérisé en ce que** l'étape d'activation d'un système de mise en sécurité comprend l'activation automatique d'un système empêchant le démarrage du véhicule.

6. Procédé selon la revendication 1, **caractérisé en ce que** l'étape d'activation d'un système de mise en sécurité comprend l'activation automatique d'un système de purge du ou des filtres à carburant.

7. Procédé selon la revendication 1, **caractérisé en ce que** l'étape d'activation automatique d'un système de mise en sécurité comprend l'activation d'un système permettant le by-pass des filtres post-traitement d'échappement.

8. Procédé selon l'une quelconque des revendications 1 à 7, **caractérisé en ce que** le système de mise en sécurité comporte des moyens d'autodiagnostic pour assurer le bon fonctionnement du système ou le cas échéant pour informer l'utilisateur, l'EOBD et le contrôle moteur.

9. Procédé selon l'une quelconque des revendications 1 à 8, **caractérisé en ce que** l'étape de diagnostic de la dégradation de la nature du carburant comprend une étape d'adressage d'au moins une table comprenant des valeurs de critères représentant la nature, le type et le niveau de dégradation du carburant, à destination du système de mise en sécurité.

10. Procédé selon l'une quelconque des revendications 1 à 9, **caractérisé en ce que** le système d'analyse comprend au moins un microanalyseur (8) permettant la mesure des interactions entre des radiations électromagnétiques et les molécules composant le carburant, ledit microanalyseur étant implanté dans le circuit carburant (1) comprenant le système de remplissage (3), le réservoir (2), les pompes (5), les filtres à carburant (6), et les circuits d'alimentation du moteur (4) et un circuit retour (7) vers le réservoir.

11. Procédé selon la revendication10, **caractérisé en ce qu'**il prévoit d'utiliser un microanalyseur spectroscopique (8)proche infrarouge.agencé pour effectuer des mesures dans les régions spectrales comprises entre 780 nm et 2500 nm.

12. Procédé selon l'une quelconque des revendications 1 à 11, **caractérisé en ce qu'**il comprend une étape de stockage des informations du type et du niveau de dégradation de la nature du carburant de façon à former un historique.

## Patentansprüche

1. Verfahren zur Sicherung der Organe des Triebwerks eines mit einem Wärmemotor ausgerüsteten Fahrzeugs vor oder während seiner Startphase im Anschluss an eine Verschlechterung der Art des im Tank (2) enthaltenen Kraftstoffs und das Versorgungssystem des Motors mit Kraftstoff, wobei das genannte Verfahren eine Diagnosestufe des Typs und des Umfangs der Verschlechterung der Art des Kraftstoffs umfasst, wobei die genannte Stufe auf der Messung der Interaktionen zwischen einer elektromagnetischen Strahlung und den den Kraftstoff bildenden Molekülen besteht, wobei die genannte Messung durch ein Analysesystem realisiert wird, und eine Aktivierungsstufe eines Sicherungssystems (13) der Organe des Triebwerks in Abhängigkeit von den Ergebnissen der Analysestufe, wobei die Messung der Interaktionen zwischen den elektromagnetischen Strahlungen und den den Kraftstoff bildenden Molekülen aus einer spektroskopischen Infrarot-Analyse und / oder einer spektroskopischen UV-Analyse und / oder einer spektroskopischen RMN-Analyse besteht, wobei die Werte der Absorbierungen, Übertragungen, Widerspiegelungen oder Ausgaben, die bei jeder ausgewählten Strahlenlänge gemessen werden, mit einer oder mehreren Spektrumhülle(n) oder Referenzpunkten derart verglichen werden, dass das Vorhandensein einer Verschlechterung der Art des Kraftstoffs und genauer gesagt des Typs und des Niveaus dieser Verschlechterung bestimmt wird.

2. Verfahren gemäß Anspruch 1, **dadurch gekennzeichnet, dass** das Sicherungssystem ein visuelles oder akustisches Alarmsystem des Nutzers ist.

3. Verfahren gemäß Anspruch 1, **dadurch gekennzeichnet, dass** das Sicherungssystem ein aktives oder passives, das Motorsteuergerät informierendes System ist.

4. Verfahren gemäß Anspruch 3, **dadurch gekennzeichnet, dass** das aktive Sicherungssystem direkt oder indirekt auf die Parameter des Kraftstoffkreislaufs, die Einspritzparameter des Kraftstoffs, die Verbrennungsparameter, die Nachbearbeitungsparameter und / oder die Startparameter des Fahrzeugs einwirkt.

5. Verfahren gemäß Anspruch 1, **dadurch gekennzeichnet, dass** die Aktivierungsstufe eines Sicherungssystems die automatische Aktivierung eines den Start des Fahrzeugs verhindernden Systems umfasst.

6. Verfahren gemäß Anspruch 1, **dadurch gekennzeichnet, dass** die Aktivierungsstufe eines Sicherungssystems die automatische Aktivierung eines Entleerungssystems des oder der Kraftstofffilter umfasst.

7. Verfahren gemäß Anspruch 1, **dadurch gekennzeichnet, dass** die automatische Aktivierungsstufe eines Sicherungssystems die Aktivierung eines den By-Pass der Nachbearbeitungsfilter des Auspuffs umfasst.

8. Verfahren gemäß Anspruch 1 bis 7, **dadurch gekennzeichnet, dass** das Sicherungssystem Autodiagnosemittel zur Gewährleistung des einwandfreien Betriebs des Systems oder ggf. zur Information des Nutzers, zum EOBD und zur Motorsteuerung umfasst.

9. Verfahren gemäß Anspruch 1 bis 8, **dadurch gekennzeichnet, dass** die Diagnosestufe der Verschlechterung der Art des Kraftstoffs eine Adressierstufe von wenigstens einer Tabelle umfasst, die Kriterienwerte umfasst, die die Art, den Typ und das Verschlechterungsniveau des Kraftstoffs umfasst, die für das Sicherungssystem bestimmt ist.

10. Verfahren gemäß Anspruch 1 bis 9, **dadurch gekennzeichnet, dass** das Analysesystem wenigstens ein Mikroanalysegerät (8) umfasst, das die Messung der Interaktionen zwischen elektromagnetischen Strahlungen und den den Kraftstoff bildenden Molekülen erlaubt, wobei das genannte Mikroanalysegerät im Kraftstoffkreislauf (1) eingebaut ist, der das Befüllsystem (3), den Tank (2), die Pumpen (5), die Kraftstofffilter (6) und die Versorgungskreisläufe des Motors (4) und einen Rückführungskreislauf (7) zum Tank umfasst.

11. Verfahren gemäß Anspruch 10, **dadurch gekennzeichnet, dass** er die Nutzung eines nahen spektroskopischen Infrarot-Mikroanalysegeräts (8) umfasst, das zur Durchführung der Messungen in den zwischen 780 nm und 2500 nm inbegriffenen spektralen Bereichen angeordnet ist.

12. Verfahren gemäß Anspruch 1 bis 11, **dadurch gekennzeichnet, dass** es eine Speicherstufe der Angaben des Typs und des Niveaus der Verschlechterung der Art des Kraftstoffs derart umfasst, dass eine Historie gebildet wird.

## Claims

1. A method for shutting down in emergency the components of the drive train of a vehicle equipped with a heat engine, before or during the of start-up phase thereof following a deterioration of the quality of the fuel contained in the tank (2) and the fuel feed system of the engine, said method comprising a step of diagnosing the type and extent of the deterioration of the quality of the fuel, said step being based on the measurement of the interactions between an electromagnetic radiation and the constituent molecules of the fuel, said measurement being performed by an analysis system, and a step of activating a system (13) for shutting down in emergency the components of the drive train as a function of the results of the step of analyzing, with the measurement of the interactions between the electromagnetic radiation and the constituent molecules of the fuel being an infrared spectroscopic analysis and/or an ultraviolet spectroscopic analysis and/or a NMR spectroscopic analysis, with the values of the absorbance, transmittance, reflectance and diffusions measured at each selected wavelength being compared to one or more spectrum envelope(s) or reference points in order to determine the existence of a degradation of the quality of the fuel, and more exactly the type and level of this degradation.

2. A method according to claim 1, **characterized in that** the emergency shutdown system is a user visual or audible alarm system.

3. A method according to claim 1, **characterized in that** the emergency shutdown system is an active or passive system informing the engine ECU.

4. A method according to claim 3, **characterized in that** the active emergency shutdown system directly or indirectly acts on the fuel system parameters, the fuel injection parameters, the combustion parameters, the aftertreatment parameters and/or the vehicle starting parameters.

5. A method according to claim 1, **characterized in that** the step of activating an emergency shutdown system comprises the automatic activation of a system preventing the starting of the vehicle.

6. A method according to claim 1, **characterized in that** the step of activating the emergency shutdown system comprises the automatic activation of a fuel filter(s) purging system.

7. A method according to claim 1, **characterized in that** the step of automatic activation of an emergency shutdown system includes the activation of a system enabling to by-pass the exhaust aftertreatment filters.

8. A method according to any one of claims 1 to 7, **characterized in that** the emergency shutdown system comprises self-diagnostic means for ensuring the proper operation of the system or, if appropriate, for informing the user, the EOBD and the engine control.

9. A method according to any one of claims 1 to 8, **characterized in that** the step of diagnosing the deterioration of the quality of the fuel comprises a step of sending at least one table comprising values of criteria representing the nature, the type and the level of degradation of the fuel system, to the emergency shutdown system.

10. A method according to any one of claims 1 to 9, **characterized in that** the analysis system comprises at least one microanalyzer (8) for measuring the interactions between the electromagnetic radiations and the constituent molecules of the fuel, with said microanalyzer being located in the fuel circuit (1) comprising the filling system (3), the tank (2), the pumps (5), the fuel filters (6) and the engine fuel supply circuits (4) and a return circuit (7) to the tank.

11. A method according to claim 10, **characterized in that** it provides to use a near infrared spectroscopic microanalyzer (8) so arranged as to make measurements in the spectral regions between 780nm and 2,500nm.

12. A method according to any one of claims 1 to 11, **characterized in that** it comprises a step of storing information on the type and level of degradation of the quality of the fuel to create a background.
